# FASCICULE DE BREVET EUROPEEN

(11) **EP 2 173 366 B1**
(45) Date de publication et mention de la délivrance du brevet: **22.05.2013**
(21) Numéro de dépôt: 08827349.5
(22) Date de dépôt: 01.07.2008
(51) Int. Cl.: A61K 35/64, A61P 25/16

(54) **UTILISATION DE VENIN D'ABEILLE POUR TRAITER LA MALADIE DE PARKINSON**
VERWENDUNG VON BIENENGIFT ZUR BEHANDLUNG VON MORBUS PARKINSON
USE OF BEE VENOM FOR TREATING PARKINSON'S DISEASE

(30) Priorité: 02.07.2007 FR 0704754; 02.07.2007 FR 0706697
(43) Date de publication de la demande: 14.04.2010
(73) Titulaire: Assistance Publique - Hôpitaux de Paris, 75004 Paris (FR)
(72) Inventeur: HARTMANN, Andréas, F-75003 Paris (FR); BONNET, Anne-Marie, F-75013 Paris (FR); SCHÜPBACH, Michael, 75005 Paris (FR)
(74) Mandataire: Barny, Luc
(86) Numéro de dépôt international: PCT/FR2008/000937
(87) Numéro de publication internationale: WO 2009/022067

(56) Documents cités:
- KRIVOPALOV-MOSCVIN I V: "Apitoxinotherapy in the treatment of Parkinson's disease" INTERNET ARTICLE, [Online] XP002470214 Extrait de l'Internet: URL:http://www.api-centre.ru/apitoxy/index _e.html> [extrait le 2006-10-13]
- LUPU ALEXANDRA: "Apitherapy - The Bee Venom Therapy" INTERNET ARTICLE 15 août 2006 (2006-08-15), XP002549609 Extrait de l'Internet: URL:http://web.archive.org/web/20060815154 445/http://news.softpedia.com/news/Apither apy-The-Bee-Venom-Therapy-30597.shtml> [extrait le 2009-10-09]
- WULFF H ET AL: "Modulators of small- and intermediate-conductance calcium-activated potassium channels and their therapeutic indications" CURRENT MEDICINAL CHEMISTRY, BENTHAM SCIENCE PUBLISHERS BV, BE, vol. 14, no. 13, juin 2007 (2007-06), pages 1437-1457, XP002470707 ISSN: 0929-8673
- LIÉGEOIS J-F ET AL: "Modulation of small conductance calcium-activated potassium (SK) channels: a new challenge in medicinal chemistry." CURRENT MEDICINAL CHEMISTRY APR 2003, vol. 10, no. 8, avril 2003 (2003-04), pages 625-647, XP009078299 ISSN: 0929-8673
- DILLY SEBASTIEN ET AL: "Identification of a pharmacophore of SKCa channel blockers" JOURNAL OF ENZYME INHIBITION AND MEDICINAL CHEMISTRY, vol. 20, no. 6, décembre 2005 (2005-12), pages 517-523, XP009085532 ISSN: 1475-6366
- HALLWORTH NICHOLAS E ET AL: "Apamin-sensitive small conductance calcium-activated potassium channels, through their selective coupling to voltage-gated calcium channels, are critical determinants of the precision, pace, and pattern of action potential generation in rat subthalamic nucleus neurons in vitro." THE JOURNAL OF NEUROSCIENCE : THE OFFICIAL JOURNAL OF THE SOCIETY FOR NEUROSCIENCE 20 AUG 2003, vol. 23, no. 20, 20 août 2003 (2003-08-20) , pages 7525-7542, XP002268345 ISSN: 1529-2401
- 'Essai clinique - N° EudraCT 2009-016702-16' ANSM Extrait de l'Internet: <URL:https://icrepec.ansm.sante.fr/Public/e ssaiDesc.php> [extrait le 2012-10-30]
- 'Bee Venom as a Neuroprotective Agent in Parkinson's Disease' 05 Novembre 2012, pages 1 - 2

### SEQUENCE LISTING

<110> ASSISTANCE PUBLIQUE - HOPITAUX DE PARIS HARTMANN, Andréas BONNET, Anne-Marie
<120> MEDICAMENT POUR LE TRAITEMENT DE LA MALADIE DE PARKINSON
<130> CHN/im-F1020/28PCT1
<160> 1
<170> PatentIn version 3.3
<210> 1
   <211> 18
   <212> PRT
   <213> Apis mellifera
<400> 1
   Cys Asn Cys Lys Ala Pro Glu Thr Ala Leu Cys Ala Arg Arg Cys Gln
   1               5                   10                  15
   Gln His

## Description

L'invention concerne l'utilisation de venin d'abeille dans la fabrication d'un médicament pour la restauration et /ou la protection des neurones, et/ou le traitement symptomatique à long terme, dans la maladie de Parkinson.

La maladie de Parkinson est un désordre qui affecte les cellules nerveuses, ou neurones, dans une partie du cerveau, la substance noire pars compacta, qui contrôle le mouvement musculaire. Dans la maladie de Parkinson, les neurones, qui produisent de la dopamine, meurent ou ne fonctionnent pas normalement.

Or, c'est entre autre la dopamine produite par les neurones qui envoie les signaux qui permettent d'initier et de coordonner les mouvements.

La cause de l'endommagement des cellules nerveuses n'est pas connue.

La maladie de Parkinson touche généralement des personnes âgées d'environ soixante ans mais peut débuter plus tôt.

L'akinésie est le symptôme majeur de la maladie de Parkinson. Ce symptôme se définit principalement comme étant un trouble d'initiation du mouvement qui amène le patient à diminuer progressivement son activité motrice. Pour plusieurs auteurs le terme d'akinésie est utilisé de façon plus large, et inclut aussi la diminution de quantité de mouvement (hypokinésie), le ralentissement dans l'exécution des mouvements (bradykinésie) et la perte de capacité à exécuter des mouvements automatiques. Aussi, les patients parkinsoniens présentent une augmentation du tonus musculaire caractéristique (rigidité de type « roue dentée »). Finalement, un tremblement de repos (4-8 Hz) est présent dans environ 2/3 des cas. Lorsque les symptômes s'amplifient, les personnes atteintes de la maladie de Parkinson ont des difficultés à marcher, à parler ou à réaliser des tâches simples. Elles peuvent également avoir des troubles dépressifs, des troubles du sommeil, ainsi que des troubles cognitifs et dysautonomiques.

Il est généralement reconnu que les symptômes de la maladie de Parkinson apparaissent seulement lorsque 50% des neurones dopaminergiques nigraux sont détruits. Outre ces 50% de neurones détruits, 15 à 20% sont dits silencieux, c'est-à-dire qu'ils restent morphologiquement intacts mais ne produisent plus ou très peu de dopamine.

Les médicaments utilisés à l'heure actuelle ne permettent que de soulager, certes considérablement, les symptômes de la maladie de Parkinson mais ne permettent pas de stopper l'évolution de la maladie et encore moins de restaurer le fonctionnement des neurones endommagés.

Les médicaments utilisés actuellement sont principalement la L-dopa dans ses diverses formes, ainsi que les agonistes dopaminergiques. La L-dopa est transformée en dopamine dans les neurones dopaminergiques par la dopa-décarboxylase. Ces médicaments produisent une variété d'effets secondaires périphériques, notamment des hypotensions et des nausées. Plus grave, après 5 à 10 ans de traitement, la pulsatilité de l'administration de ces molécules en plusieurs prises par jour - en contraste avec le relargage constant de dopamine physiologique- induit des fluctuations motrices généralement très invalidantes. Aussi, il est important de noter que ces molécules agissent purement de manière symptomatique et ne ralentissent pas le processus dégénératif.

L'invention vise à pallier les inconvénients des médicaments utilisés dans le traitement de la maladie de Parkinson en proposant un médicament qui permet non seulement de protéger les neurones non endommagés mais également de restaurer la fonction des neurones dits silencieux tout en ne provoquant pas les effets secondaires dus à l'administration de L-dopa. Aussi, à plus court terme, l'invention devrait permettre l'obtention d'un effet symptomatique durable.

La fréquence d'injection de cette dose unitaire dépendra également du patient et du stade de sa maladie.

Ainsi, en début de traitement, une injection toutes les semaines est appropriée. Puis en fonction de l'évolution de l'état du patient, la fréquence d'injections pourra être d'une injection toutes les six semaines.

Dans tous les cas, ceci est un avantage important par rapport à la L-dopa qui doit être administrée quotidiennement et à plusieurs prises par jour. Dans un mode de mise en oeuvre préféré de l'utilisation de venin d'abeilles la dose unitaire est à injecter sous-cutanément toutes les unes à six semaines pour soulager les symptômes et/ou restaurer et/ou protéger les neurones de patients atteints de la maladie de Parkinson.

Autrement dit, le venin d'abeille entier pourra être utilisé.

En effet, le venin d'abeille est un produit naturel bien maîtrisé, déjà utilisé en thérapeutique pour la désensibilisation des sujets allergiques au venin d'abeille.

Mais surtout, le venin d'abeille contient de l'apamine en une quantité d'environ 3% en poids, par rapport au poids total du venin d'abeille.

La composition du venin de *Apis mellifera*, l'abeille domestique européenne commune, est reportée au tableau I suivant:

**Tableau I**

| **Classe de composés** | **Composés** | **Pourcentage par rapport à la masse sèche** |
|---|---|---|
| Enzymes | Phospholipase A2 | 10-12 |
| | Hyaluronidase | 1-2 |
| | Phosphomonoestérase acide | 1,0 |
| | a-D-glucosidase | 0,6 |
| | Lysophospholipase | 1,0 |
| Polypeptides | Mélittine | 40-50 |
| | Mélittine-F | 0,01 |
| | Apamine | 3 |
| | Peptide dégranulant les mastocytes | 2 |
| | Sécapine | 0,5 |
| | Tertiapine | 0,1 |
| | Inhibiteur de protéase | - |
| | Procamine A et B | 1,4 |
| Basses masses moléculaires | Histamine | 0,66-1,6 |
| | Dopamine | 0,13-1 |
| | Noradrénaline | 0,1-0,7 |

Cette composition peut varier, faiblement, d'une espèce d'abeille à l'autre.

Ainsi, l'invention concerne l'utilisation de venin d'abeille, en une quantité comprise entre 33 microgrammes et 330 microgrammes, pour la fabrication d'une dose unitaire, pour injection sous-cutanée, toutes les une à six semaines, d'un médicament pour soulager les symptômes et/ou restaurer et/ou protéger les neurones de patients atteints de la maladie de Parkinson.

La quantité de venin d'abeille à utiliser dans la fabrication de cette dose unitaire dépendra du patient à traiter et en particulier de son poids.

Cette quantité de venin d'abeille est généralement comprise entre 33 microgrammes et 330 microgrammes inclus, de préférence entre 66 microgrammes et 165 microgrammes, le plus préférablement de 100 à 110 microgrammes inclus.

L'invention sera mieux comprise et d'autres avantages et caractéristiques de celle-ci apparaîtront plus clairement à la lecture de la description qui suit et qui est faite en référence à un exemple non limitatif et purement illustratif.

### Exemple:

Un patient de 82 kilos, à un stade parkinsonien avancé (15 ans de maladie) a été traité, suite à une réaction allergique au venin d'abeille par une injection mensuelle de 110 microgrammes de venin d'abeille.

A la suite de chaque injection, le score moteur tel que déterminé sur l'échelle de notation de la maladie de Parkinson unifiée (United Parkinson's Disease Rating Scale -UPDRS III) s'est amélioré de 70% en quelques heures et lui a permis ensuite de supprimer entièrement son traitement par L-dopa durant deux à quatre semaines.

Dans tous les cas, pendant les deux semaines suivant l'injection et précédant l'injection suivante de venin d'abeille, son traitement par L-dopa a diminué de 50% par rapport à la période précédant l'injection de venin d'abeille.

Ceci montre que l'apamine possède une activité aussi bien symptomatique que neuroprotectrice mais surtout qu'elle possède une activité neurorestauratrice dans la maladie de Parkinson.

Bien entendu, la dose unitaire contenant de l'apamine ou de venin d'abeille peut être utilisée en combinaison avec d'autres thérapies.

Cette dose unitaire de venin d'abeille peut également être administrée par une autre voie que la voie sous-cutanée. Cependant pour des raisons de disponibilité de la quantité totale administrée, la voie par injection sous-cutanée reste préférée.

## Revendications

1. Utilisation de venin d'abeille, en une quantité comprise entre de 33 à 330 microgrammes inclus, pour la fabrication d'une dose unitaire pour injection sous-cutanée, toutes les une à six semaines, d'un médicament pour soulager les symptômes et/ou restaurer et/ou protéger les neurones de patients atteints de la maladie de Parkinson.

2. Utilisation selon la revendication 1, **caractérisée en ce que** la quantité de venin d'abeille est comprise entre 66 et 165 microgrammes.

3. Utilisation selon la revendication 1 ou 2, **caractérisée en ce que** la quantité de venin d'abeille est de 110 microgrammes.

## Patentansprüche

1. Verwendung von Bienengift in einer Menge im Bereich zwischen einschließlich 33 und 330 Mikrogramm zur Herstellung einer Dosierungseinheit zur subkutanen Injektion eines Medikaments alte ein bis sechs Wochen, um die Symptome von Patienten, die an der Parkinson-Krankheit leiden, zu lindern und/oder deren Neutronen zu restaurieren und/oder zu schützen.

2. Verwendung nach Anspruch 1, **dadurch gekennzeichnet, dass** die Menge an Bienengift im Bereich zwischen 66 und 165 Mikrogramm liegt.

3. Verwendung nach Anspruch 1 ober 2, **dadurch gekennzeichnet, dass** die Menge an Bienengift bei 110 Miprogramm liegt.

## Claims

1. Use of bee venom, in an amount between 33 and 330 micrograms inclusive, for producing a unit dose for subcutaneous injection, every one to six weeks, of a drug for relieving the symptoms and/or restoring and/or protecting the neurons of patients with Parkinson's disease.

2. Use according to claim 1, **characterised in that** the amount of bee venom is between 66 and 165 micrograms.

3. Use according to either claim 1 or claim 2, **characterised in that** the amount of bee venom is 110 micrograms.
